Europäisches Patentamt

European Patent Office

Office européen des brevets

(19)

(11) **EP 0 734 004 A1**

(12) # DEMANDE DE BREVET EUROPEEN

(43) Date de publication:
**25.09.1996 Bulletin 1996/39**

(51) Int Cl.⁶: **G08B 21/00, A61G 7/05**

(21) Numéro de dépôt: **96400565.6**

(22) Date de dépôt: **19.03.1996**

(84) Etats contractants désignés:
**CH DE ES GB IT LI**

(30) Priorité: **20.03.1995 FR 9503207**

(71) Demandeur: **Saubatjou, Christian**
**65290 Juillan (FR)**

(72) Inventeur: **Saubatjou, Christian**
**65290 Juillan (FR)**

(74) Mandataire: **Bonnetat, Christian**
**CABINET BONNETAT**
**23, Rue de St. Pétersbourg**
**75008 Paris (FR)**

(54) **Dispositif de veille assistée pour un meuble, notamment un lit**

(57) -La présente invention concerne un dispositif de veille assistée (1) pour un meuble (L), en particulier un lit, destiné à permettre à une personne (P) de se coucher ou de s'asseoir.

- Selon l'invention, ledit dispositif (1) comporte au moins un bras (2) monté de façon pivotante sur ledit meuble (L) et agencé latéralement audit meuble (L) de manière à barrer la descente dudit meuble (L), et un système électrique associé audit bras pivotant (2) et susceptible d'émettre un signal d'alarme ; et le pivotement dudit bras (2) par une personne (P) qui a pris place sur ledit meuble (L) entraîne l'émission dudit signal d'alarme par ledit système électrique.

FIG.1

EP 0 734 004 A1

## Description

La présente invention concerne un dispositif de veille assistée pour un meuble, notamment un lit, sur lequel une personne peut se coucher ou s'asseoir.

Bien que la présente invention puisse s'appliquer à de nombreux meubles, comme par exemple un canapé ou un fauteuil, notamment un fauteuil roulant, elle est particulièrement bien adaptée à un lit, en particulier un lit d'hôpital.

On sait que certaines personnes alitées, par exemple des personnes handicapées ou des personnes excessivement agitées, en particulier à la suite d'accidents cérébraux, nécessitent une surveillance et/ou une protection efficaces et quasi-permanentes.

Pour éviter que de telles personnes alitées ne sortent ou ne tombent du lit, on fixe généralement de chaque côté de celui-ci une barrière métallique. Une telle solution a toutefois comme inconvénient de faire ressembler le lit à une cage et procure ainsi une impression désagréable d'emprisonnement. De plus, si une personne alitée essaye malgré tout de franchir ces barrières, il existe un risque important qu'elle se blesse.

Au lieu ou en plus d'une telle protection mécanique, on prévoit souvent un dispositif de surveillance qui comporte, soit une caméra dirigée sur le lit et permettant de visualiser ledit lit sur un écran de contrôle, soit un système radio qui détecte les bruits engendrés au niveau du lit et les transmet à un personnel de surveillance. Toutefois, de tels dispositifs de surveillance supposent une attention constante dudit personnel de surveillance et sont donc extrêmement fatigants, surtout si plusieurs lits doivent être surveillés simultanément, ce qui est par exemple souvent le cas dans un hôpital.

La présente invention a pour objet de remédier à ces inconvénients. Elle concerne un dispositif de veille assistée pour un meuble sur lequel une personne peut s'asseoir ou se coucher, permettant d'alerter de façon efficace un personnel de surveillance, soit d'un mouvement dangereux de cette personne, soit d'un appel à l'aide, ledit dispositif de veille assistée ne nécessitant pas une attention constante dudit personnel de surveillance.

A cet effet, ledit dispositif de veille assistée est remarquable, selon l'invention :

-   en ce qu'il comporte :

    .   au moins un bras monté de façon pivotante sur ledit meuble et agencé latéralement audit meuble de manière à barrer la descente dudit meuble, et
    .   un système électrique associé audit bras pivotant et susceptible d'émettre un signal d'alarme ; et

-   en ce que le pivotement dudit bras pivotant par une personne qui a pris place sur ledit meuble entraîne l'émission dudit signal d'alarme par ledit système électrique.

Ainsi, le pivotement dudit bras provoqué par ladite personne alitée ou assise, par exemple lorsqu'elle essaye de descendre dudit meuble, entraîne l'émission dudit signal d'alarme alertant le personnel de surveillance. Ce dispositif est très efficace puisque l'émission du signal d'alarme peut être prévue à l'endroit, ou aux endroits, où se trouve ledit personnel de surveillance.

De plus, le pivotement dudit bras peut être provoqué :

-   soit involontairement, lorsque la personne alitée ou assise bouge excessivement et/ou veut descendre du meuble,
-   soit volontairement, lorsque cette personne désire appeler de l'aide.

Comme indiqué précédemment, dans le cadre de la présente invention, ledit meuble peut correspondre en particulier à un lit, à un canapé ou à un fauteuil, notamment un fauteuil roulant, ce qui met bien en évidence la multiplicité d'utilisations possibles de ladite invention.

Le dispositif conforme à l'invention est particulièrement bien adapté au milieu hospitalier en gériatrie et pédiatrie, mais il peut également être utilisé dans un milieu familial où une personne, notamment une personne âgée, doit être surveillée continuellement ou temporairement, par exemple la nuit.

Souvent la descente dudit meuble n'est possible que d'un seul côté, par exemple dans le cas d'un lit lorsque celui-ci est disposé contre un mur. Dans ce cas, un seul bras est agencé sur ledit meuble.

En revanche, lorsque la descente dudit meuble est possible latéralement des deux côtés, ledit dispositif comporte avantageusement deux bras agencés chacun sur l'un desdits côtés.

Avantageusement, ledit bras est susceptible de pivoter dans un plan horizontal et/ou dans un plan vertical.

En outre, ledit bras est monté de préférence dans un boîtier agencé sur un montant dudit meuble, ledit boîtier renfermant au moins une partie dudit système électrique.

Dans un premier mode de réalisation avantageux, ledit bras est de forme télescopique. Ainsi, en emboîtant les différents tronçons dudit bras télescopique les uns dans les autres, on libère facilement l'accès audit meuble.

Dans un second mode de réalisation particulièrement avantageux, ledit bras pivotant est de forme cylindrique en une seule pièce et est monté dans un logement d'une pièce de fixation associée audit boîtier, ledit logement étant formé d'un trou cylindrique traversant de diamètre légèrement plus grand que le diamètre dudit bras, de manière à permettre le coulissement dudit bras dans ledit logement, et ladite pièce de fixation est munie d'une vis permettant de fixer ledit bras dans ledit logement. De plus, de préférence, ledit boîtier est monté sur ledit montant du meuble de manière à pouvoir pivoter de 90 degrés conjointement avec ledit bras dans un plan vertical, ce qui permet de libérer facilement l'accès audit meuble en pivotant ledit bras de manière à l'amener parallèlement audit montant.

De façon avantageuse, ledit signal d'alarme est différent selon que le pivotement dudit bras est effectué dans un plan horizontal ou dans un plan vertical, ce qui permet de distinguer un appel d'urgence d'un appel non urgent. Il suffit à cet effet de demander à la personne alitée ou assise de pivoter le bras dans un plan vertical lors d'un simple appel non urgent, puisque, lors d'un mouvement involontaire, donc dans un cas d'urgence, le bras est généralement pivoté dans un plan horizontal.

Selon l'invention, ledit signal d'alarme peut être un signal sonore et/ou un signal lumineux, adaptés bien entendu à l'environnement et aux conditions d'utilisation de l'invention.

Par ailleurs, de façon avantageuse, un indicateur lumineux permettant d'indiquer le déclenchement d'un signal d'alarme peut être agencé à proximité du meuble, pour prévenir la personne alitée ou assise d'un tel déclenchement.

Les figures du dessin annexé feront bien comprendre comment l'invention peut être réalisée. Sur ces figures, des références identiques désignent des éléments semblables.

La figure 1 montre en perspective un lit muni d'un dispositif conforme à l'invention.

La figure 2 montre en perspective la partie mécanique du dispositif conforme à l'invention, fixée sur le lit sur la figure 1.

La figure 3 est une coupe transversale de la partie mécanique de la figure 2.

La figure 4 illustre schématiquement le système électrique du dispositif conforme à l'invention.

La figure 5 montre en perspective un boîtier permettant de centraliser les signaux d'alarme d'une pluralité de dispositifs conformes à l'invention.

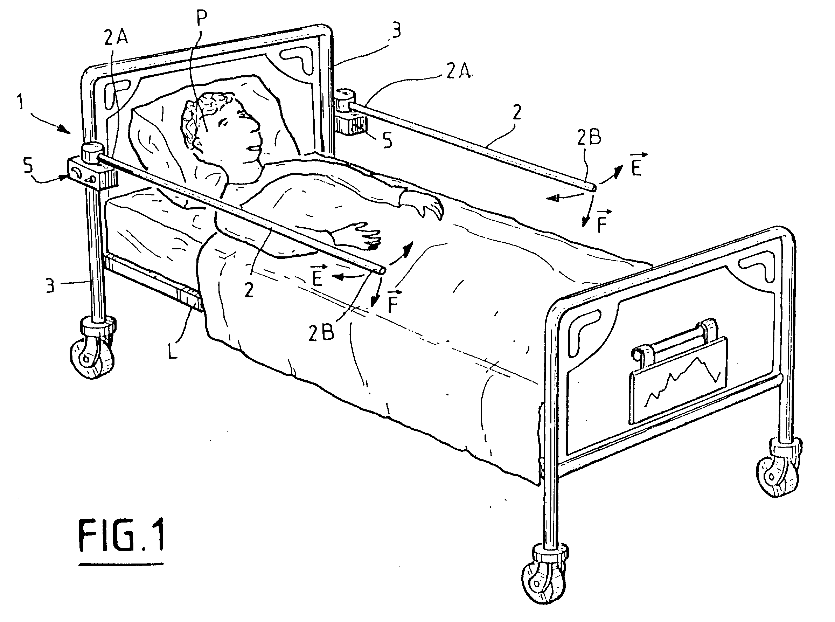

Le dispositif de veille assistée 1 conforme à l'invention est destiné à être monté sur un meuble L, sur lequel une personne P peut se coucher ou s'asseoir, tel que représenté sur la figure 1.

Bien que la présente invention puisse ainsi être appliquée à de très nombreux meubles, comme par exemple un canapé ou un fauteuil, notamment un fauteuil roulant, on décrira l'invention ci-après en référence à un lit L, plus précisément un lit d'hôpital.

Ledit dispositif 1 est particulièrement bien adapté à la surveillance de personnes alitées excessivement agitées, par exemple à la suite d'un accident cérébral, en permettant d'alerter un personnel de surveillance, surtout comme on le verra ci-après lorsque la personne alitée P essaye de sortir dudit lit L.

A cet effet, selon l'invention, ledit dispositif 1 comporte deux bras longitudinaux identiques 2 agencés respectivement de part et d'autre du lit L de manière à barrer la descente dudit lit L.

Chacun desdits bras 2 est monté de façon pivotante, par l'une 2A de ses extrémités 2A et 2B, sur un montant 3 du lit L, tel que précisé ci-après, et est disposé de façon horizontale, latéralement le long dudit lit L.

Bien entendu, dans le cas où la descente du lit L n'est possible que d'un seul côté, par exemple lorsque le lit est disposé contre un mur ou lorsqu'il comporte une barrière fixe à l'un des côtés, le dispositif 1 comporte un seul bras 2 agencé du côté où la descente reste possible.

Dans la suite de la description, on se référera uniquement à un seul bras 2, étant entendu que, dans le cas où le dispositif 1 comporte deux bras, le second bras est formé de façon identique à celui décrit ci-après.

Le bras 2 peut pivoter :

- d'une part, dans un plan horizontal, tel qu'indiqué par une flèche double $\vec{E}$ pour l'extrémité libre 2B dudit bras 2 ; et
- d'autre part, dans un plan vertical vers le bas, tel qu'illustré par une flèche $\vec{F}$.

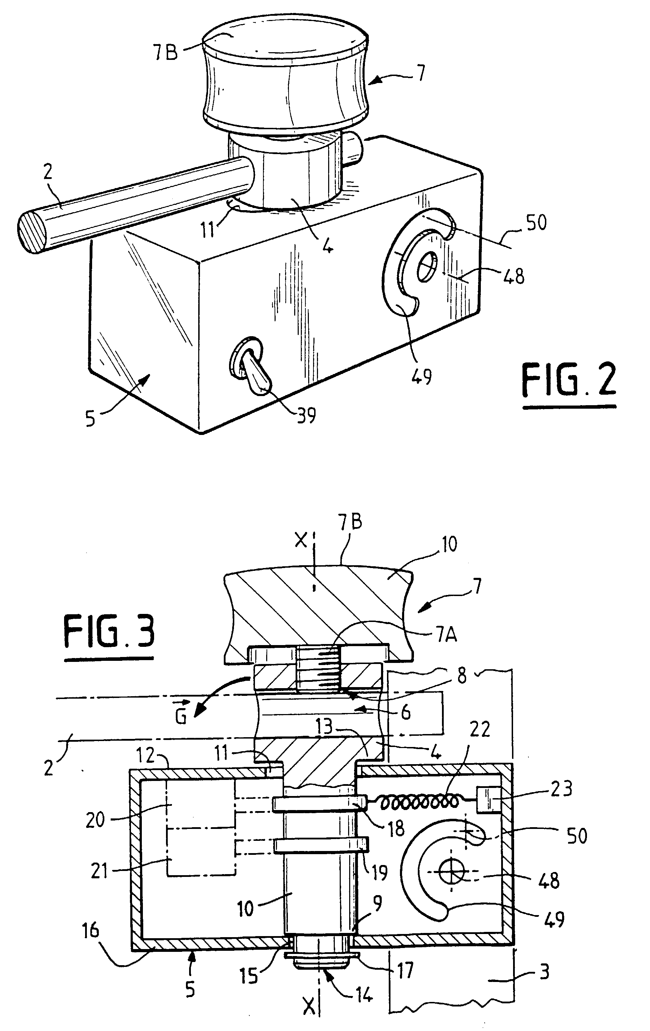

A cet effet, comme représenté sur la figure 2, ledit bras 2 de forme cylindrique est monté dans une pièce de fixation 4 qui est solidaire d'un boîtier 5, de forme parallélépipédique, fixé sur le montant 3 du lit L.

La pièce de fixation 4 présente une ouverture traversante transversale 6 représentée sur la figure 3, de forme cylindrique, servant de logement audit bras 2. Ledit logement 6 est dimensionné de manière à permettre le coulissement dudit bras 2. Ce dernier peut être bloqué dans ledit logement 6 au moyen d'une vis 7, dont le fût fileté 7A coopère avec un perçage fileté 8 de la pièce de fixation 4 et dont la tête 7B peut être tournée manuellement.

Ladite pièce de fixation 4 comporte un tronc cylindrique 10 d'axe X-X qui, dans la position montée de la figure 3, traverse un trou 11 pratiqué dans la face supérieure 12 dudit boîtier 5 et ladite pièce de fixation 4 repose de façon lâche par un épaulement annulaire 13 sur ladite face supérieure 12. L'extrémité 14 dudit tronc 10, opposée à l'épaulement 13, traverse un trou 15 dans la face inférieure 16 dudit boîtier 5. La pièce de fixation 4 est maintenue dans ledit boîtier 5, d'une part, au moyen de l'épaulement annulaire 13 qui repose sur la face 12 du boîtier 5 et d'un épaulement annulaire 9 qui repose sur la face 16 et, d'autre part, au moyen d'un élément de fixation 17 de forme annulaire et de diamètre plus grand que le diamètre du trou 15, entourant l'extrémité 14 du tronc 10 à l'extérieur du boîtier 5.

La pièce de fixation 4 comporte en outre deux cames 18 et 19, de type connu, qui sont formées autour du tronc 10 l'une au-dessus de l'autre, par exemple par usinage, et qui coopèrent respectivement avec des moyens de commutation 20 et 21 représentés schématiquement sur la figure 3 et précisés ci-après.

Par ailleurs, comme on peut le distinguer sur les figures 2 et 3, le trou 11 est de forme oblongue. Il est plus grand en direction longitudinale du bras 2, dans la position montée de la figure 2, qu'en direction transversale. Une telle réalisation permet un déplacement de la pièce de fixation 4 dans le sens indiqué par une flèche $\vec{G}$, qui correspond au déplacement de l'extrémité 2B du bras 2 dans le sens de la flèche $\vec{F}$ de la figure 1.

De plus, un ressort de rappel 22 est fixé, d'une part, à la came 18 et, d'autre part, à une saillie 23 solidaire du boîtier 5, ledit ressort de rappel 22 exerçant une force de rappel dans le cas d'un déplacement de la pièce de fixation 4 selon le sens $\vec{G}$.

On notera de plus que le trou 15 présente un diamètre plus grand que l'extrémité 14 du tronc 10, de manière à permettre le déplacement précité. A cet effet, ledit trou 15 peut également être formé de façon oblongue.

Selon l'invention, la came 18 et le moyen de commutation associé 20 sont formés de sorte que le déplacement de la pièce de fixation 4, de sa position de repos de la figure 3, dans le sens de la flèche $\vec{G}$, actionne ledit moyen de commutation 20.

La pièce de fixation 4 peut en outre tourner autour de l'axe X-X. La came 19 et le moyen de commutation associé 21 sont, quant à eux, formés de sorte qu'une telle rotation autour dudit axe X-X, à partir de la position de repos de la figure 3, actionne ledit moyen de commutation 21.

Par ailleurs, dans un but de perfectionnement, ledit boîtier 5 peut comporter une ouverture non représentée destinée à permettre un accès facile à l'intérieur dudit boîtier 5, par exemple lors d'une réparation, et ledit bras 2 peut comporter une bague externe fluorescente non représentée permettant de voir ledit bras la nuit.

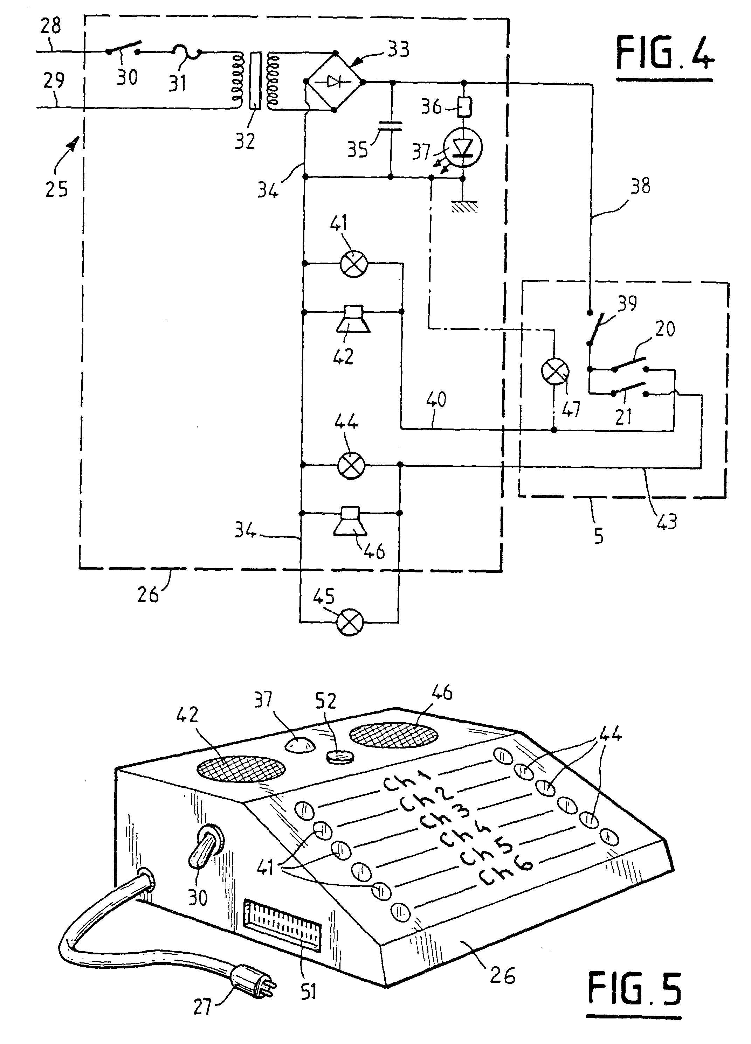

En plus de la partie mécanique décrite ci-dessus, le dispositif 1 conforme à l'invention comporte également un système électrique 25 représenté schématiquement sur la figure 4, dont une partie est intégrée dans le boîtier 5 représenté en traits interrompus, et dont l'autre partie est indépendante et peut être intégrée pour l'essentiel, dans un mode de réalisation avantageux de l'invention, dans un boîtier 26 représenté sur la figure 5.

Le dispositif 1 peut être relié au secteur par une prise 27 à deux fils 28 et 29.

A l'un 28 desdits fils, sont reliés successivement un interrupteur 30 destiné à mettre sous tension ledit dispositif 1 et un fusible de protection 31.

Lesdits fils 28 et 29 sont ensuite reliés à un transformateur abaisseur 32, qui abaisse la tension du secteur et délivre à sa sortie une tension adaptée au dispositif 1, par exemple 12V.

A la sortie dudit transformateur abaisseur 32 est raccordé un pont de diodes 33 :

- dont l'une des sorties est reliée à une liaison 34 amenée à la masse, à laquelle est raccordée une borne d'un condensateur 35, et

- dont l'autre sortie est reliée à l'autre borne dudit condensateur 35.

Audit condensateur 35 est relié en parallèle un ensemble formé d'une résistance 36 et d'une diode électroluminescente 37 montées en série. Cette diode électroluminescente 37 est activée lorsque l'interrupteur 30 est fermé et que le dispositif 1 est sous tension.

En outre, une liaison 38, dont l'une des extrémités est reliée à un point entre le condensateur 35 et la résistance 36, est raccordée par son autre extrémité à un interrupteur 39 monté sur le boîtier 5.

En série à cet interrupteur 39, sont montés les deux systèmes de commutation 20 et 21 reliés ensemble et représentés sous forme d'interrupteurs sur la figure 4.

A ces interrupteurs sont associés des systèmes de maintien automatique du courant électrique du type connu non représentés et permettant, lorsque les systèmes de commutation 20 et 21 sont actionnés, de fournir continuellement du courant électrique à des moyens d'avertissement décrits ci-après, jusqu'à ce que l'interrupteur 39 soit fermé, ceci même si lesdits interrupteurs 20 et 21 sont ouverts auparavant, soit automatiquement, par exemple par le ressort de rappel 22 qui remet le système de commutation 20 dans sa position initiale, soit manuellement, par le pivotement du bras 2 par exemple par la personne P en sens inverse du sens de pivotement initial.

Audit système de commutation 20 est raccordée en outre une liaison 40 qui est reliée à l'une des bornes, d'une part, d'une source lumineuse 41 et, d'autre part, d'un générateur de son 42, les autres bornes de ladite source lumineuse 41 et dudit générateur de son 42 étant raccordées à la liaison 34.

Le système de commutation 21, quant à lui, est relié à une liaison 43 qui est raccordée à deux sources lumineuses 44 et 45 et à un générateur de son 46, également raccordés à la liaison 34.

La fermeture du système de commutation 20 déclenche donc la source lumineuse 41 et le générateur de son 42, tandis que la fermeture du système de commutation 21 déclenche les sources lumineuses 44 et 45 et le générateur de son 46.

Bien entendu, dans le cas où le dispositif 1 comporte deux bras 2, ledit système électrique 25 comporte de plus deux systèmes de commutation non représentés, associés au second bras, identiques respectivement aux systèmes de commutation 20 et 21 du premier bras et reliés, de façon identique auxdits systèmes de commutation 20 et 21, respectivement aux liaisons 40 et 43 de manière à pouvoir déclencher les générateurs de son 42 et 46 et les sources lumineuses 41, 44 et 45.

On précise à présent le fonctionnement du dispositif 1 conforme à l'invention décrit précédemment.

Lorsque la personne alitée P s'agite ou veut sortir du lit L et qu'elle pousse le bras 2 latéralement, celui-ci pivote dans un plan horizontal, ce qui provoque la rotation de la pièce de fixation 4 autour de l'axe X-X. Ladite rotation actionne le système de commutation 21 qui déclenche les sources lumineuses 44 et 45 et le générateur de son 46, susceptibles d'avertir un personnel de surveillance.

Pour plus d'efficacité, dans le cas d'un hôpital, la source lumineuse 45 peut être installée dans le couloir, par exemple au-dessus de la porte de la chambre dans laquelle se trouve le lit L, et la source lumineuse 44 et le générateur de son 46 sont montés sur le boîtier 26 installé par exemple dans une salle de surveillance.

Bien entendu, dans le cadre de l'invention, un seul de ces différents moyens d'avertissement 44, 45 et 46 peut être suffisant.

L'opération précédente initialisée par le pivotement du bras 2 dans un plan horizontal peut également être déclenchée volontairement par la personne alitée P, lorsqu'en cas d'urgence elle veut appeler de l'aide.

Dans le cas plus fréquent où la personne alitée P veut appeler de l'aide, mais que ce n'est pas urgent, elle peut pousser le bras 2 vers le bas pour le faire pivoter dans le sens de la flèche $\vec{F}$, ce qui provoque le déplacement de la pièce de fixation 4 dans le sens de la flèche $\vec{G}$ et, ainsi, l'actionnement du système de commutation 20 qui déclenche la source lumineuse 41 et le générateur de son 42, avertissant le personnel de surveillance.

Comme, selon l'invention, ledit personnel de surveillance est en mesure de distinguer les différentes sources lumineuses et les différents générateurs de son, dont les sons émis peuvent être engendrés différemment à cet effet, il est en mesure de savoir si l'appel est un appel d'urgence ou non.

Le mode de réalisation décrit précédemment à titre d'exemple est un mode de réalisation particulièrement avantageux présentant de nombreux perfectionnements. Bien entendu, dans le cadre de la présente invention, il est également possible :

- de ne pas différencier l'alarme entre un pivotement horizontal et un pivotement vertical,
- d'utiliser un seul moyen d'avertissement, une source lumineuse ou un générateur de son,
- de ne permettre le pivotement du bras que dans un seul plan, de préférence dans ce cas dans le plan horizontal.

Dans un autre mode de réalisation non représenté, le dispositif 1 comporte un moyen, par exemple sous forme d'un système à bride, permettant de limiter le débattement latéral de l'extrémité 2B du bras 2, ce qui permet de conférer audit dispositif une sécurité proche de celle des barrières fixes connues, avec en plus une fonction d'appel si on appuie sur ledit bras 2.

Le dispositif 1 peut également comporter un indicateur lumineux, à savoir une source lumineuse 47 ou sonore représentée sur la figure 4, reliée aux liaisons 34 et 40 et indiquant l'actionnement du moyen de commutation 20. Cette source lumineuse 47 peut par exemple être agencée sur le boîtier 5 ou à proximité du lit L pour avertir la personne alitée P lorsqu'elle a déclenché le dispositif 1. Une telle source lumineuse peut également être associée au moyen de commutation 21.

Par ailleurs, le boîtier 5 est fixé sur le montant 3 du lit L de manière à pouvoir pivoter autour d'un axe 48. Ledit boîtier 5 comporte de plus une lumière 49, en forme d'arc de cercle, qui est destinée à coopérer avec un pion non représenté d'axe 50, fixé sur ledit montant 3.

Ainsi, lorsque le personnel de surveillance veut s'occuper de la personne alitée P, il effectue les opérations suivantes :

- il met hors service le dispositif 1 au moyen de l'interrupteur 39,
- il dévisse la vis 7, fait coulisser le bras 2 vers l'arrière et revisse la vis 7,
- il fait pivoter le boîtier 5 à peu près de 90° de sorte que le bras 2 est parallèle au montant 3 et permet un accès libre à ladite personne alitée P.

Dans un autre mode de réalisation non représenté, ledit bras 2 peut être de forme télescopique de manière à rendre possible l'accès à la personne alitée en emboîtant les tronçons dudit bras télescopique les uns dans les autres.

La présente invention peut être mise en oeuvre aussi bien dans une collectivité, comme un hôpital ou une maison de retraite, que dans un milieu familial, par exemple pour la surveillance d'une personne handicapée.

Dans le cas où plusieurs lits situés dans différentes chambres sont munis du dispositif 1 conforme à l'invention, les signaux d'alarme peuvent être centralisés dans le boîtier 26 représenté sur la figure 5.

Sur ce boîtier 26, on a agencé un seul générateur de son 42 et 46 et une seule source lumineuse 41 et 44 par chambre Chl à Ch6. Ainsi, lors d'une alarme, le personnel de surveillance sait immédiatement de quelle chambre celle-ci provient.

Le boîtier 26 comporte à cet effet une prise multiple 51 permettant la liaison aux différents dispositifs 1. Sur ledit boîtier 26, on a également représenté un potentiomètre 52 destiné à ajuster l'intensité du signal sonore émis par les générateurs de son 42 et 46.

**Revendications**

1. Dispositif de veille assistée pour un meuble (L), en particulier un lit, qui est destiné à permettre à une personne (P), notamment un malade, de se coucher ou de s'asseoir, et sur lequel au moins un bras (2) est monté latéralement de façon pivotante en barrant la descente dudit meuble (L), caractérisé en ce qu'il comporte un système électrique (25) associé audit bras pivotant (2) et susceptible d'émettre un signal d'alarme, de sorte que le pivotement dudit bras pivotant (2) par ladite personne (P) qui a pris place sur ledit meuble (L) entraîne l'émission dudit signal d'alarme par ledit système électrique (25).

2. Dispositif selon la revendication 1, la descente dudit meuble (L) étant possible latéralement des deux côtés,
caractérisé en ce qu'il comporte deux bras (2) agencés chacun sur l'un desdits côtés.

3. Dispositif selon l'une quelconque des revendications précédentes,
caractérisé en ce que ledit bras (2) est susceptible de pivoter dans un plan horizontal.

4. Dispositif selon l'une quelconque des revendications précédentes,
caractérisé en ce que ledit bras (2) est susceptible de pivoter dans un plan vertical.

5. Dispositif selon l'une quelconque des revendications précédentes,
caractérisé en ce que ledit bras (2) est monté dans un boîtier (5) agencé sur un montant (3) dudit meuble (L), ledit boîtier (5) renfermant au moins une partie dudit système électrique (25).

6. Dispositif selon l'une quelconque des revendications précédentes,
caractérisé en ce que ledit bras est de forme télescopique.

7. Dispositif selon la revendication 5,
caractérisé en ce que ledit bras pivotant (2) est de forme cylindrique en une seule pièce et est monté dans un logement (6) d'une pièce de fixation (4) associée audit boîtier (5), ledit logement (6) étant formé d'un trou cylindrique traversant de diamètre légèrement plus grand que le diamètre dudit bras (2), de manière à permettre le coulissement dudit bras (2) dans ledit logement (6), et en ce que ladite pièce de fixation (4) est munie d'une vis (7) permettant de fixer ledit bras (2) dans ledit logement (6).

8. Dispositif selon l'une des revendications 5 ou 7,
caractérisé en ce que ledit boîtier (5) est monté sur ledit montant (3) du meuble (L) de manière à pouvoir pivoter de 90 degrés, conjointement avec ledit bras (2), dans un plan vertical.

9. Dispositif selon les revendications 3 et 4,
caractérisé en ce que ledit signal d'alarme est différent selon que le pivotement dudit bras (2) est effectué dans un plan horizontal ou dans un plan vertical.

10. Dispositif selon l'une quelconque des revendications précédentes,

caractérisé en ce que ledit signal d'alarme est un signal sonore.

11. Dispositif selon l'une quelconque des revendications précédentes,
caractérisé en ce que ledit signal d'alarme est un signal lumineux.

12. Dispositif selon l'une quelconque des revendications précédentes,
caractérisé en ce qu'un indicateur lumineux (47) permettant d'indiquer le déclenchement d'un signal d'alarme est agencé à proximité dudit meuble (L).

FIG.1

FIG.2

FIG.3

FIG.4

FIG.5

**Office européen des brevets**

# RAPPORT DE RECHERCHE EUROPEENNE

Numero de la demande

EP 96 40 0565

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | US-A-4 612 679 (L. D. MITCHELL)<br>* colonne 1, ligne 44 - colonne 2, ligne 25; figure 1 *<br>* colonne 10, ligne 40 - ligne 54 *<br>--- | 1,2 | G08B21/00<br>A61G7/05 |
| Y | US-A-4 183 015 (W. D. DREW ET AL)<br>* colonne 3, ligne 1 - colonne 4, ligne 27; figure 3 *<br>--- | 1,2 | |
| P,A | GB-A-2 284 147 (T. R. FERRIS)<br>* abrégé; figure 1 *<br>--- | | |
| A | US-A-4 067 005 (J. LEVY ET AL)<br>* abrégé; figure 1 *<br>--- | | |
| A | US-A-3 875 356 (J. V. HEIM ET AL)<br>* abrégé; figure 1 *<br>----- | | |

**DOMAINES TECHNIQUES RECHERCHES (Int.Cl.6)**

G08B
A61G
A47C

Le présent rapport a été établi pour toutes les revendications

| Lieu de la recherche | Date d'achèvement de la recherche | Examinateur |
|---|---|---|
| BERLIN | 12 Juin 1996 | Breusing, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)